# EUROPEAN PATENT SPECIFICATION

(11) **EP 4 616 808 B1**
(45) Date of publication and mention of the grant of the patent: **22.04.2026**
(21) Application number: 25160275.1
(22) Date of filing: 26.02.2025
(51) Int. Cl.: A61B 6/03, A61B 6/40, A61B 6/42, A61B 6/58

(54) **PHOTON COUNTING X-RAY CT APPARATUS AND CALIBRATION DATA ACQUISITION METHOD**
PHOTONENZÄHLENDE RÖNTGEN-CT-VORRICHTUNG UND KALIBRIERUNGSDATENERFASSUNGSVERFAHREN
APPAREIL DE TOMODENSITOMÉTRIE À RAYONS X À COMPTAGE DE PHOTONS ET PROCÉDÉ D'ACQUISITION DE DONNÉES D'ÉTALONNAGE

(30) Priority: 11.03.2024 JP 2024036560
(43) Date of publication of application: 17.09.2025
(73) Proprietor: FUJIFILM Corporation, Tokyo 106-8620 (JP)
(72) Inventor: KOJIMA, Shinichi, Tokyo (JP); YOKOI, Kazuma, Tokyo (JP); TAKAHASHI, Isao, Tokyo (JP); TERAMOTO, Fuyuhiko, Tokyo (JP)
(74) Representative: Dehns Germany Partnerschaft mbB

(56) References cited:
- US-A1- 2021 212 646
- US-A1- 2022 395 248
- US-A1- 2023 233 164

## Description

### BACKGROUND OF THE INVENTION

### 1. Field of the Invention

The present disclosure relates to a photon counting X-ray CT apparatus and a calibration data acquisition method thereof.

### 2. Description of the Related Art

A photon counting type X-ray computed tomography (CT) apparatus uses a photon counting type detector as a detector thereof. The photon counting type detector can discriminate the energy of incident radiation photons and count the number of incident radiation photons for each energy region (bin). Consequently, in the photon counting type X-ray CT apparatus, it is possible to obtain a medical image in which materials having different compositions are discriminated, such as a medical image in which, for example, an iodine contrast agent used in angiography and calcified plaques in blood vessels are discriminated. In order to obtain a medical image in which materials are discriminated in this manner, it is necessary to acquire, in advance, a relationship between photon energy and an output in a case in which a phantom composed of a combination of a plurality of base materials, which are materials having known compositions and thicknesses, is measured by the photon counting type detector, as calibration data for each detection element. For example, JP2022-190306A discloses a phantom and a radiation imaging apparatus, and a calibration method for a photon counting type detector that are capable of shortening a time required to acquire configuration data even with a large irradiation field. JP2022-190306A discloses that a phantom is used by combining a first base material and a second base material, and that a phantom of the first base material is driven by a patient table and a phantom of the second base material is driven by a dedicated driving unit, in order to individually drive the phantom of the first base material and the phantom of the second base material. Document US 2022/395248 A1 describes a photon counting X-ray CT apparatus and two phantoms used in calibration of the photon counting detector.

### SUMMARY OF THE INVENTION

As the phantom used to acquire the calibration data, a plurality of base materials having known compositions and thicknesses, for example, a combination of two base materials, that is, a first base material and a second base material, are used. In a case in which the calibration data is acquired using a plurality of phantoms obtained by changing thicknesses of the base materials to be combined, it is necessary to replace the phantoms for the acquisition of projection DG175976 data. Meanwhile, for example, in a case in which these plurality of phantoms are configured as a single phantom, such as a step-shaped phantom disclosed in the related art of JP2022-190306A, the phantom becomes large and is difficult to handle.

According to the technology disclosed in JP2022-190306A, by individually driving the phantom of the first base material and the phantom of the second base material, it is possible to reduce a user's tasks for acquiring the calibration data. However, this requires a new driving unit for moving the phantom of the second base material.

The present invention also utilizes a mechanism provided in the photon counting X-ray CT apparatus for the movement of the phantom, thereby minimizing an additional mechanism to the apparatus and reducing the user's tasks, enabling the acquisition of calibration data.

According to an embodiment of the present invention, there is provided a photon counting X-ray CT apparatus comprising: an X-ray tube; a filter; a filter drive mechanism that drives the filter; a detector that includes a photon counting type detection element; a gantry to which the X-ray tube and the filter drive mechanism, and the detector are attached so as to face each other with an opening portion interposed therebetween; and a computing device, in which a second phantom of a second base material, which has different thickness portions, is installed on the filter, the computing device moves the filter using the filter drive mechanism such that X-rays from the X-ray tube are transmitted through a desired thickness portion of the second phantom, and the computing device radiates X-rays from the X-ray tube in a state in which a first phantom of a first base material, which has a smaller linear attenuation coefficient than the second base material, is inserted into the opening portion such that X-rays from the X-ray tube are transmitted through the first phantom of the first base material, and acquires projection data based on a detection value output by the detector.

Provided are a photon counting X-ray CT apparatus and a calibration data acquisition method that enable acquisition of calibration data by minimizing an additional mechanism to the apparatus and reducing a user's tasks. Other objects and novel features will become apparent from the description and the accompanying drawings of the present specification.

### BRIEF DESCRIPTION OF THE DRAWINGS

Fig. 1 is an overall configuration diagram of a photon counting X-ray CT apparatus.
Fig. 2A is a diagram illustrating calibration of a photon counting type detector.
Fig. 2B is a diagram illustrating calibration of the photon counting type detector.
Fig. 3 is a diagram illustrating a calibration data acquisition method of Example 1.
Fig. 4 is a diagram schematically showing a configuration of a bowtie filter on which a second phantom is disposed.
Fig. 5 is a diagram illustrating a calibration data acquisition method of Example 2.
Fig. 6A is a diagram illustrating a calibration data acquisition method of Example 3.
Fig. 6B is a diagram illustrating the calibration data acquisition method of Example 3.
Fig. 7 is a diagram illustrating the calibration data acquisition method of Example 3.

### DESCRIPTION OF THE PREFERRED EMBODIMENTS

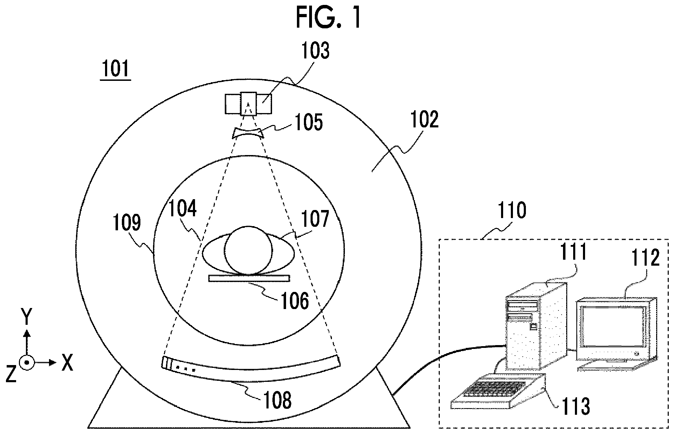

Fig. 1 shows an overall configuration diagram of a photon counting X-ray CT apparatus 101. Herein, a horizontal direction of a paper surface is defined as an X axis, a vertical direction is defined as a Y axis, and a direction orthogonal to an XY plane is defined as a Z axis. The X-ray CT apparatus 101 comprises, as a main configuration, a gantry 102, an X-ray tube 103, a bowtie filter 105, a patient table 106, a detector 108, and a console 110.

A subject 107 is placed on the patient table 106 and is disposed in an opening portion 109 provided in the gantry 102. X-rays 104 radiated from the X-ray tube 103 are formed into a beam shape suitable for a size of the subject 107 by the bowtie filter 105, emitted to the subject 107, transmitted through the subject 107, and then detected by the detector 108. The X-ray tube 103 and the detector 108 are attached to the gantry 102 so as to face each other with the subject 107 interposed therebetween and are rotated around the subject 107 by a rotation driving unit of the gantry 102. By repeatedly performing the X-ray irradiation from the X-ray tube 103 and the X-ray measurement by the detector 108 with the rotation by the rotation driving unit, projection data at various projection angles is acquired.

The console 110 comprises a computing device 111, a display device 112, and an input device 113. The acquired projection data is subjected to image reconstruction processing by the computing device 111, thereby generating and displaying a tomographic image of the subject 107 on the display device 112. Additionally, in a case in which the projection data is acquired while the patient table 106 with the subject 107 placed thereon and the gantry 102 move relative to each other in a Z axis direction, a volume image of the subject 107 is generated. An X-ray dose emitted from the X-ray tube 103, a rotation speed of the gantry 102, and a relative movement speed between the gantry 102 and the patient table 106 are set based on scan conditions input by an operator via the input device 113.

The detector 108 is configured by disposing a plurality of detection elements in an arc shape centered on the X-ray focal point of the X-ray tube 103. The detection element is a photon counting type detection element that measures X-ray energy, which is energy of incident X-ray photons, and outputs a detection value corresponding to the X-ray energy. In the X-ray CT apparatus 101 comprising such a photon counting type detector, the X-ray energy spectrum related to the projection data of the subject 107 can be acquired, thereby enabling the generation of a medical image in which materials having different compositions are discriminated or medical images divided into a plurality of energy components.

In addition, the computing device 111 has the same hardware configuration as a general computer device, comprises a central processing unit (CPU), a memory, a non-volatile storage device such as a hard disk drive (HDD), and performs control of each unit and correction processing on the projection data and the like.

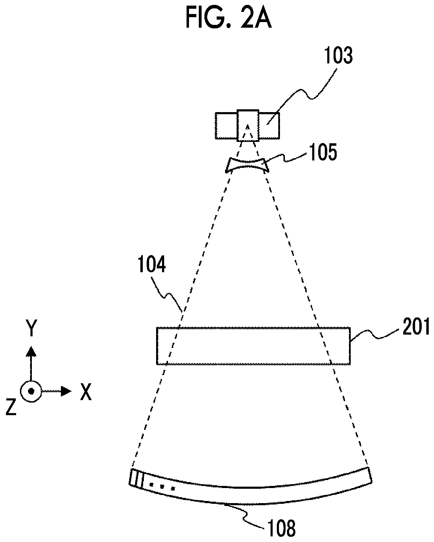

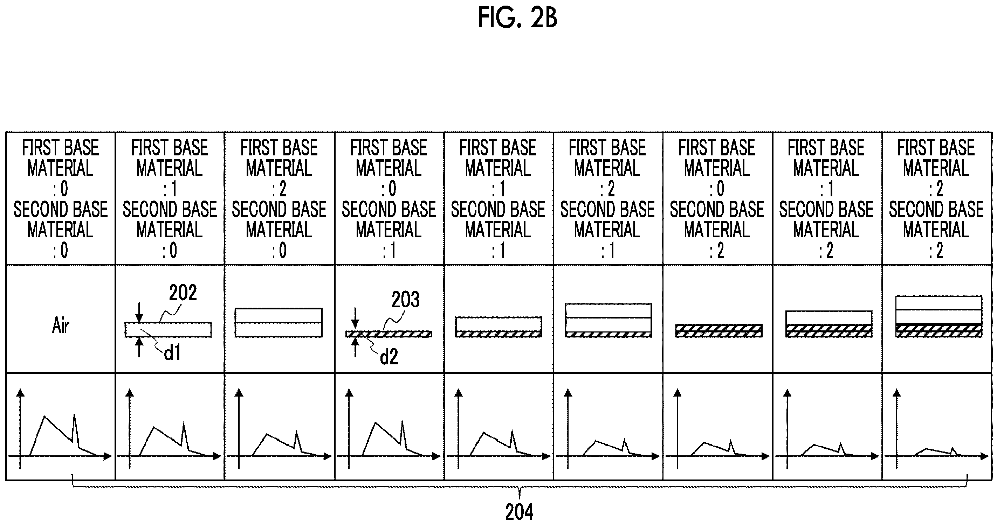

The calibration of the photon counting type detector will be described using Figs. 2A and 2B. In the calibration of the photon counting type detector, as shown in Fig. 2A, a phantom 201 is disposed between the X-ray tube 103 and the detector 108, and the projection data is acquired and used as calibration data. For the phantom 201, a plurality of base materials having known compositions and thicknesses, for example, a combination of two base materials, that is, a first base material 202 and a second base material 203, are used. The base material is not particularly limited, but in a case in which a person is assumed as a subject, acrylic or polyethylene, which is a material having a linear attenuation coefficient close to that of water, can be selected as the first base material 202, and tin or titanium, which is a material having a linear attenuation coefficient close to that of bone or a contrast agent, can be selected as the second base material 203. Additionally, as the phantom 201, a plurality of combinations of the first base material 202 and the second base material 203, each having different thicknesses, are prepared. For example, in a case in which the first base material 202 has J types of thicknesses and the second base material 203 has K types of thicknesses, a phantom that is a combination of J × K types of base materials is used, and the photon energy spectrum is acquired for each detection element for each combination.

In the example of Fig. 2B, since J = 3 and K = 3, nine types of photon energy spectra are shown as calibration data 204. The acquired calibration data 204 is stored in a storage unit of the computing device 111 and is used to calibrate the projection data of the subject 107. In this example, a plurality of sets of the first base material 202 and the second base material 203, each having the same thickness, are combined to obtain the base materials having different thicknesses, but base materials having different thicknesses may be prepared. In either case, it is desirable that the thicknesses of the base materials that constitute the phantom 201 are integer multiples of thinnest thicknesses d1 and d2. By doing so, the computational load for calibrating the projection data can be reduced.

### Example 1

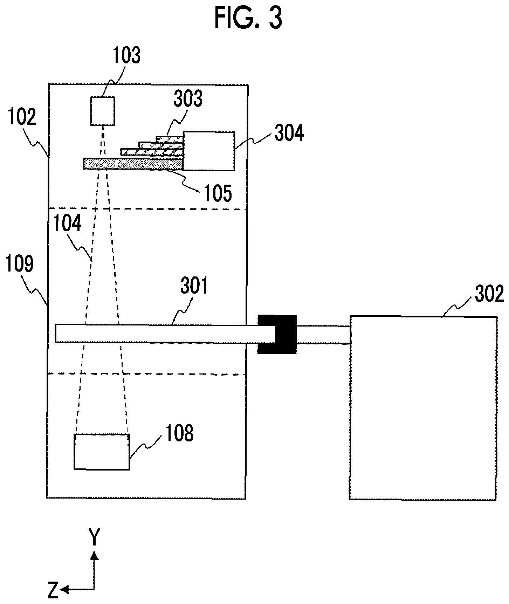

A calibration data acquisition method of a photon counting type detector of Example 1 will be described using Fig. 3. In the present example, a phantom used to calibrate the photon counting type detector is a combination of a first phantom 301 of the first base material having a small linear attenuation coefficient and a second phantom 303 of the second base material having a large linear attenuation coefficient, and the second phantom 303 is installed on the bowtie filter 105. During calibration data acquisition, the plate-like first phantom 301 having a desired thickness is inserted into the opening portion 109 using a carriage 302, the second phantom 303 installed on the bowtie filter 105 is moved by a filter drive mechanism 304, and the X-rays 104 from the X-ray tube 103 are transmitted through the second phantom 303 having a desired thickness.

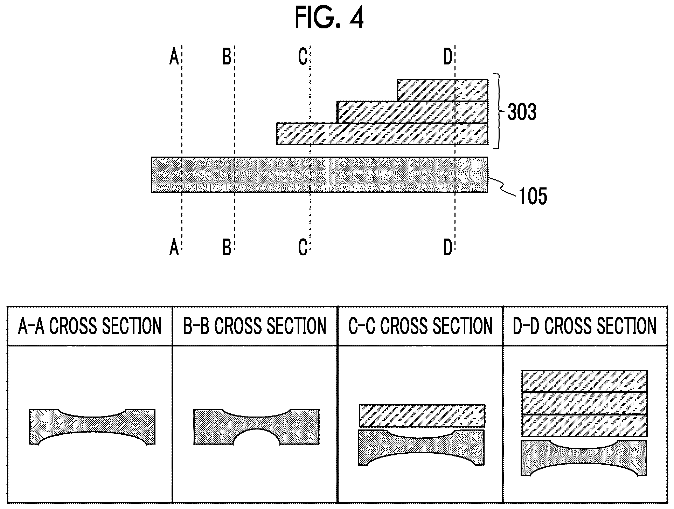

Fig. 4 schematically shows a configuration of the bowtie filter 105 on which the second phantom 303 is disposed. The bowtie filter is provided in order to equalize the dose of X-rays 104 reaching the detector 108 and to suppress unnecessary exposure caused by X-rays reaching a region outside a field of view (FOV). Therefore, it is desirable that the shape of the bowtie filter is changed according to the FOV. In that respect, the bowtie filter 105 includes differently shaped portions as shown in an A-A cross section and a B-B cross section, enabling the filter drive mechanism 304 to adjust the position of the bowtie filter 105 through which the X-rays 104 are transmitted. The present example also utilizes this mechanism for adjusting the thickness of the second phantom 303.

The second phantom 303 having different thickness portions is installed on the bowtie filter 105. In Fig. 4, a stack obtained by staking the second base material having the same thickness with a shifted position is used as the second phantom 303. That is, by using the second base material with one layer for a C-C cross section and the second base material with three layers for a D-D cross section, the thickness of the second phantom 303 is varied. A fixing unit of the second phantom 303 to the bowtie filter 105 is not limited as long as it does not interfere with a region through which the X-rays 104 are transmitted through the bowtie filter 105 and/or the second phantom 303. In a case in which the second base material is tin, the thickness of the second base material with one layer can be on the order of 0.01 mm. Therefore, even in a case in which the second phantom 303 is fixed to the filter drive mechanism 304, there is no adverse effect on the driving of the bowtie filter 105 by the filter drive mechanism 304. In addition, here, an example has been shown in which the second phantom 303 is installed on an X-ray tube 103 side with respect to the bowtie filter 105, but the second phantom 303 may be installed on an opening portion 109 side with respect to the bowtie filter 105.

In the example of Fig. 4, the C-C cross section and the D-D cross section have the same filter shape as the A-A cross section. The filter drive mechanism 304 can move both the bowtie filter 105 and the second phantom 303 at once, thereby enabling the acquisition of the calibration data while changing the thickness of the first phantom 301 and the thickness of the second phantom 303 with respect to the bowtie filter 105 in the A-A cross section. Meanwhile, the calibration data in a case in which the thickness of the first phantom 301 and the thickness of the second phantom 303 are changed with respect to the bowtie filter 105 in the B-B cross section can be obtained as follows. First, a change in the X-rays due to a difference in the filter shape is calculated from the projection data in a case in which the X-rays are transmitted through the B-B cross section to change the thickness of the first phantom 301 and the projection data in a case in which the X-rays are transmitted through the A-A cross section to change the thickness of the first phantom 301. The projection data in a case in which the thickness of the first phantom 301 and the thickness of the second phantom 303 are changed with respect to the bowtie filter 105 in the B-B cross section can be calculated using the calculated change in the X-rays due to the filter shape and the projection data for each thickness of the second phantom 303 with respect to the bowtie filter 105 in the A-A cross section. Considering the nonlinear response of the detector 108 dependent on the X-ray dose, the calibration data in the B-B cross section is obtained.

In Example 1, the thickness of the second phantom 303 can be changed by the filter drive mechanism 304, and the user need only perform replacement work of the first phantom 301 into the opening portion 109. As a result, the user's workload required to acquire the calibration data can be reduced.

Here, an example has been shown in which the second phantom 303 is installed on the bowtie filter 105, but the second phantom 303 may be installed on a filter other than the bowtie filter 105. For example, the second phantom 303 may be installed on a low-energy X-ray removal filter. The low-energy X-ray removal filter is a filter for changing the spectrum of the X-rays 104, and the same effect can be obtained even in a case in which the second phantom 303 is installed on the low-energy X-ray removal filter and the thickness of the second phantom 303 is changed by a drive mechanism thereof. The same applies to the following examples.

Additionally, in Fig. 4, an example has been shown in which the second phantom 303 and the bowtie filter 105 are moved together by the filter drive mechanism 304 and the calibration data in the B-B cross section in which the second phantom 303 does not overlap is obtained through computational operation, but the second phantom 303 and the bowtie filter 105 may be driven separately. In this case, the calibration data in the B-B cross section can be obtained through actual measurement. Further, the second phantom 303 may be configured to overlap with different thicknesses in an A-A cross-sectional shape portion and a B-B cross-sectional shape portion of the bowtie filter 105, allowing for the calibration data in the B-B cross section to be obtained through actual measurement.

### Example 2

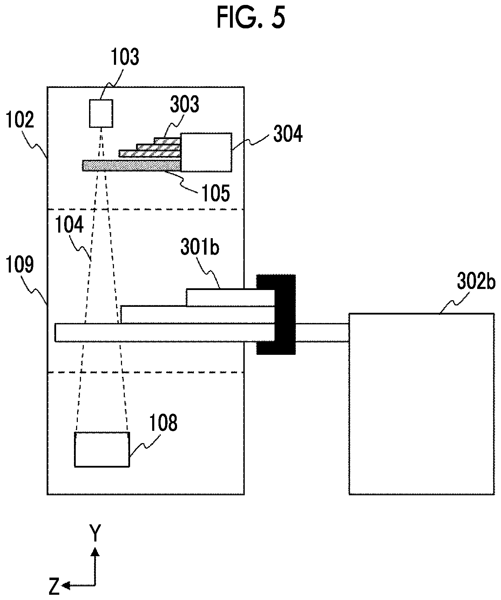

A calibration data acquisition method of a photon counting type detector of Example 2 will be described using Fig. 5. In Example 1, in order to change the thickness of the first phantom 301, replacement work of the first phantom 301 having a different thickness into the opening portion 109 occurs. In Example 2, by inserting a first phantom 301b having different thickness portions into the opening portion 109 using a carriage 302b comprising a phantom drive mechanism (not shown), the user's workload can be reduced.

In Fig. 5, a stack obtained by stacking the plate-like first base material having the same thickness with a shifted position is used as the first phantom 301b. The user controls the filter drive mechanism 304 and the phantom drive mechanism of the carriage 302b in conjunction with each other to change the thickness of each of the first phantom 301b and the second phantom 303 through which the X-rays 104 are transmitted, thereby changing the combination of the thickness of the first phantom 301b and the thickness of the second phantom 303 to acquire the calibration data. As a result, the user's workload required to acquire the calibration data can be further reduced.

### Example 3

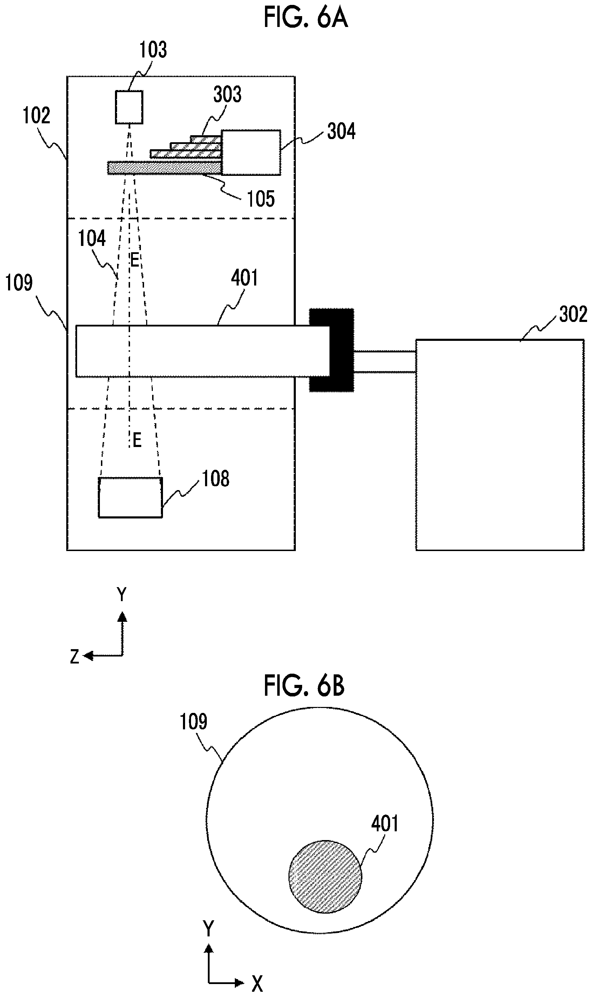

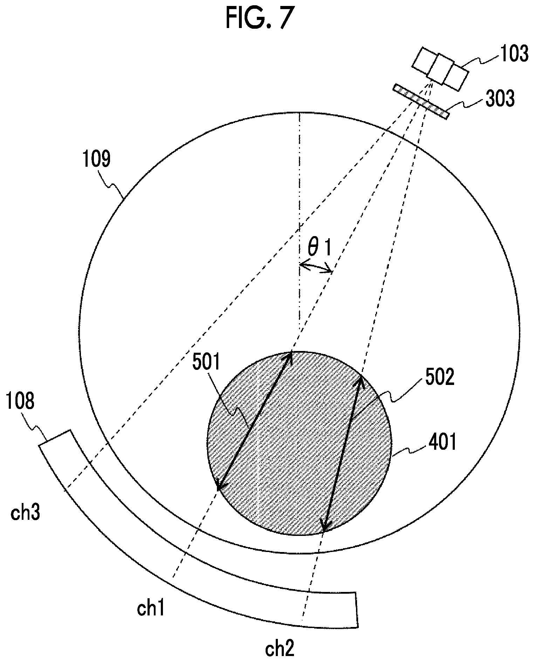

A calibration data acquisition method of a photon counting type detector of Example 3 will be described using Figs. 6A, 6B, and 7. Fig. 6B shows an E-E cross section (a cross section parallel to the XY plane) in a state in which a first phantom 401 is inserted into the opening portion 109. The first phantom 401 is inserted into the opening portion 109 by the carriage 302 so as not to overlap with a rotation center of the gantry 102. The first phantom 401 has a cylindrical shape, and a diameter of a cross section of the first phantom 401 is smaller than a radius of the opening portion 109.

In Example 3, the calibration data is acquired while the gantry 102 is rotated. Fig. 7 shows a state of the X-ray tube 103, the second phantom 303, the first phantom 401, and the detector 108 in a case in which the calibration data is acquired. Since the shape, the size, the position in the opening portion 109 of the first phantom 401 are known, a length of a portion where a straight line connecting the X-ray tube 103 and each detection element of the detector 108 overlaps with the first phantom 401 is calculated as a transmission length of the first phantom 401. Fig. 7 shows a transmission length 501 in the detection element of ch1 and a transmission length 502 in the detection element of ch2 in a case in which a projection angle is θ1. The transmission length in the detection element of ch3 where the X-rays reach without being transmitted through the first phantom 401 is zero.

In this case, the user can change the thickness of the second phantom 303 using the filter drive mechanism 304, rotate the gantry 102, and repeatedly perform processing of acquiring the calibration data, thereby changing the combination of the transmission length of the first phantom 401 and the thickness of the second phantom 303 to acquire the calibration data. As a result, the user's workload required to acquire the calibration data can be further reduced.

It should be noted that an example of the first phantom having a cylindrical shape has been shown here, but the cross section of the first phantom is not limited to a circle and may be a polygon. In this case, a polygon having acute internal angles is desirable. In this case as well, the circumscribed circle of the polygon, which is the cross section of the first phantom, has a diameter smaller than the radius of the opening portion 109 and is inserted into the opening portion 109 such that the center of the circumscribed circle does not overlap with the rotation center of the gantry 102.

The present invention is not limited to the embodiments mentioned above and includes various modification examples. For example, the embodiments described above have been described in detail in order to describe the present invention in an easily understandable manner, and the present invention is not necessarily limited to those having all the configurations described above. Additionally, some of the configurations of one embodiment can be replaced with a configuration of another embodiment, and a configuration of another embodiment can be added to the configurations of one embodiment. Moreover, for some of the configurations of each embodiment, addition of other configurations, deletion, or replacement is possible.

### Explanation of References

101: photon counting X-ray CT apparatus
102: gantry
103: X-ray tube
104: X-rays
105: bowtie filter
106: patient table
107: subject
108: detector
109: opening portion
110: console
111: computing device
112 display device
113: input device
201: phantom
202: first base material
203: second base material
204: calibration data
301, 401: first phantom
302: carriage
303: second phantom
304: filter drive mechanism
501, 502: transmission length

## Claims

1. A photon counting X-ray CT apparatus (101) comprising:
an X-ray tube (103);
a filter (105);
a filter drive mechanism (304) that is configured to drive the filter (105);
a detector (108) that includes a photon counting type detection element;
a gantry (102) to which the X-ray tube (103) and the filter drive mechanism (304), and the detector (108) are attached so as to face each other with an opening portion (109) interposed therebetween; and
a computing device (111),
wherein a second phantom (303) of a second base material (203), which has different thickness portions, is installed on the filter (105),
the computing device (111) is configured to move the filter (105) using the filter drive mechanism (304) such that X-rays (104) from the X-ray tube (103) are transmitted through a desired thickness portion of the second phantom (303), and
the computing device (111) is configured to radiate X-rays (104) from the X-ray tube (103) in a state in which a first phantom (301, 401) of a first base material (202), which has a smaller linear attenuation coefficient than the second base material (203), is inserted into the opening portion (109) such that X-rays (104) from the X-ray tube (103) are transmitted through the first phantom (301, 401) of the first base material (202), and is configured to acquire projection data based on a detection value output by the detector (108).

2. The photon counting X-ray CT apparatus (101) according to claim 1,
wherein the computing device (111) is configured to acquire the projection data for each combination of a plurality of thicknesses of the first base material (202) and a plurality of thicknesses of the second base material (203), and is configured to obtain calibration data (204) of the detector (108).

3. The photon counting X-ray CT apparatus (101) according to claim 2,
wherein the second phantom (303) is a stack of the second base material (203) having the same thickness, and
a difference in thickness of the second phantom (303) is due to the number of stacked layers of the second base material (203).

4. The photon counting X-ray CT apparatus (101) according to claim 2,
wherein the first phantom (301, 401) is a stack of the first base material (202) having the same thickness, and
a difference in thickness of the first phantom (301, 401) is due to the number of stacked layers of the first base material (202).

5. The photon counting X-ray CT apparatus (101) according to claim 2,
wherein the first phantom (301, 401) is a column-shaped first base material (202) and is inserted into the opening portion (109) so as not to overlap with a rotation center of the gantry (102), and
the computing device (111) is configured to acquire the projection data while rotationally driving the gantry (102).

6. The photon counting X-ray CT apparatus (101) according to claim 5,
wherein a cross section of the first phantom (301, 401) is circular or polygonal.

7. The photon counting X-ray CT apparatus (101) according to claim 1,
wherein the filter (105) is a bowtie filter.

8. A calibration data acquisition method of a photon counting X-ray CT apparatus (101) including an X-ray tube (103), a filter (105), a filter drive mechanism (304) that drives the filter (105), a detector (108) that includes a photon counting type detection element, a gantry (102) to which the X-ray tube (103) and the filter drive mechanism (304), and the detector (108) are attached so as to face each other with an opening portion (109) interposed therebetween, and a computing device (111), the calibration data acquisition method comprising:
installing a second phantom (303) of a second base material (203), which has different thickness portions, on the filter (105);
causing the computing device (111) to move the filter (105) using the filter drive mechanism (304) such that X-rays (104) from the X-ray tube (103) are transmitted through a desired thickness portion of the second phantom (303); and
causing the computing device (111) to radiate X-rays (104) from the X-ray tube (103) in a state in which a first phantom (301, 401) of a first base material (202), which has a smaller linear attenuation coefficient than the second base material (203), is inserted into the opening portion (109) such that X-rays (104) from the X-ray tube (103) are transmitted through the first phantom (301, 401) of the first base material (202), and to acquire projection data based on a detection value output by the detector (108).

9. The calibration data acquisition method according to claim 8,
wherein the first phantom (301, 401) is a column-shaped first base material (202) and is inserted into the opening portion (109) so as not to overlap with a rotation center of the gantry (102), and
the computing device (111) acquires the projection data while rotationally driving the gantry (102).

10. The calibration data acquisition method according to claim 9,
wherein a cross section of the first phantom (301, 401) is circular or polygonal.

## Patentansprüche

1. Röntgen-CT-Vorrichtung (101) mit Photonenzählung, umfassend:
eine Röntgenröhre (103);
einen Filter (105);
einen Filterantriebsmechanismus (304), der so konfiguriert ist, dass er den Filter (105) antreibt;
einen Detektor (108), der ein Detektionselement des Photonenzählungstyps enthält;
eine Gantry (102), an der die Röntgenröhre (103) und der Filterantriebsmechanismus (304) und der Detektor (108) so angebracht sind, dass sie einander zugewandt sind, wobei ein Öffnungsabschnitt (109) dazwischen eingefügt ist; und
eine Rechenvorrichtung (111),
wobei ein zweites Phantom (303) eines zweiten Basismaterials (203), das unterschiedliche Dickenabschnitte aufweist, an dem Filter (105) installiert ist,
die Rechenvorrichtung (111) so konfiguriert ist, dass sie den Filter (105) unter Verwendung des Filterantriebsmechanismus (304) so bewegt, dass Röntgenstrahlen (104) von der Röntgenröhre (103) durch einen gewünschten Dickenabschnitt des zweiten Phantoms (303) transmittiert werden, und
die Rechenvorrichtung (111) so konfiguriert ist, dass sie Röntgenstrahlen (104) von der Röntgenröhre (103) in einem Zustand, in dem ein erstes Phantom (301, 401) eines ersten Basismaterials (202), das einen kleineren linearen Dämpfungskoeffizienten als das zweite Basismaterial (203) aufweist, in den Öffnungsabschnitt (109) so eingeführt ist, dass Röntgenstrahlen (104) von der Röntgenröhre (103) durch das erste Phantom (301, 401) des ersten Basismaterials (202) transmittiert werden, abstrahlt, und so konfiguriert ist, dass sie Projektionsdaten auf der Grundlage eines von dem Detektor (108) ausgegebenen Detektionswerts erfasst.

2. Röntgen-CT-Vorrichtung (101) mit Photonenzählung nach Anspruch 1,
wobei die Rechenvorrichtung (111) so konfiguriert ist, dass sie die Projektionsdaten für jede Kombination aus mehreren Dicken des ersten Basismaterials (202) und mehreren Dicken des zweiten Basismaterials (203) erfasst, und so konfiguriert ist, dass sie Kalibrierungsdaten (204) des Detektors (108) erhält.

3. Röntgen-CT-Vorrichtung (101) mit Photonenzählung nach Anspruch 2,
wobei das zweite Phantom (303) ein Stapel des zweiten Basismaterials (203) mit der gleichen Dicke ist, und
ein Dickenunterschied des zweiten Phantoms (303) auf die Anzahl an gestapelten Schichten des zweiten Basismaterials (203) zurückzuführen ist.

4. Röntgen-CT-Vorrichtung (101) mit Photonenzählung nach Anspruch 2,
wobei das erste Phantom (301, 401) ein Stapel des ersten Basismaterials (202) mit der gleichen Dicke ist, und
ein Dickenunterschied des ersten Phantoms (301, 401) auf die Anzahl an gestapelten Schichten des ersten Basismaterials (202) zurückzuführen ist.

5. Röntgen-CT-Vorrichtung (101) mit Photonenzählung nach Anspruch 2,
wobei das erste Phantom (301, 401) ein säulenförmiges erstes Basismaterial (202) ist und in den Öffnungsabschnitt (109) so eingeführt ist, dass es sich nicht mit einem Drehzentrum der Gantry (102) überlappt, und
die Rechenvorrichtung (111) so konfiguriert ist, dass sie die Projektionsdaten erfasst, während sie die Gantry (102) drehend antreibt.

6. Röntgen-CT-Vorrichtung (101) mit Photonenzählung nach Anspruch 5,
wobei ein Querschnitt des ersten Phantoms (301, 401) kreisförmig oder polygonal ist.

7. Röntgen-CT-Vorrichtung (101) mit Photonenzählung nach Anspruch 1,
wobei der Filter (105) ein Bowtie-Filter ist.

8. Kalibrierungsdaten-Erfassungsverfahren einer Röntgen-CT-Vorrichtung (101) mit Photonenzählung, die eine Röntgenröhre (103), einen Filter (105), einen Filterantriebsmechanismus (304), der den Filter (105) antreibt, einen Detektor (108), der ein Detektionselement des Photonenzählungstyps enthält, eine Gantry (102), an der die Röntgenröhre (103) und der Filterantriebsmechanismus (304) und der Detektor (108) so angebracht sind, dass sie einander zugewandt sind, wobei ein Öffnungsabschnitt (109) dazwischen eingefügt ist, und eine Rechenvorrichtung (111) enthält, wobei das Kalibrierungsdaten-Erfassungsverfahren umfasst:
Installieren eines zweiten Phantoms (303) eines zweiten Basismaterials (203), das unterschiedliche Dickenabschnitte aufweist, auf dem Filter (105);
Veranlassen der Rechenvorrichtung (111), den Filter (105) unter Verwendung des Filterantriebsmechanismus (304) so zu bewegen, dass Röntgenstrahlen (104) von der Röntgenröhre (103) durch einen gewünschten Dickenabschnitt des zweiten Phantoms (303) transmittiert werden; und
Veranlassen der Rechenvorrichtung (111), Röntgenstrahlen (104) von der Röntgenröhre (103) in einem Zustand, in dem ein erstes Phantom (301, 401) eines ersten Basismaterials (202), das einen kleineren linearen Dämpfungskoeffizienten als das zweite Basismaterial (203) aufweist, in den Öffnungsabschnitt (109) so eingeführt ist, dass Röntgenstrahlen (104) von der Röntgenröhre (103) durch das erste Phantom (301, 401) des ersten Basismaterials (202) transmittiert werden, abzustrahlen, und Projektionsdaten auf der Grundlage eines von dem Detektor (108) ausgegebenen Detektionswerts zu erfassen.

9. Kalibrierungsdaten-Erfassungsverfahren nach Anspruch 8,
wobei das erste Phantom (301, 401) ein säulenförmiges erstes Basismaterial (202) ist und in den Öffnungsabschnitt (109) so eingeführt ist, dass es sich nicht mit einem Drehzentrum der Gantry (102) überlappt, und
die Rechenvorrichtung (111) die Projektionsdaten erfasst, während sie die Gantry (102) drehend antreibt.

10. Kalibrierungsdaten-Erfassungsverfahren nach Anspruch 9,
wobei ein Querschnitt des ersten Phantoms (301, 401) kreisförmig oder polygonal ist.

## Revendications

1. Appareil de tomodensitométrie à rayons X à comptage de photons (101) comprenant :
un tube à rayons X (103) ;
un filtre (105) ;
un mécanisme d'entraînement de filtre (304) qui est configuré pour entraîner le filtre (105) ;
un détecteur (108) qui inclut un élément de détection de type comptage de photons ;
un portique (102) auquel le tube à rayons X (103) et le mécanisme d'entraînement de filtre (304), et le détecteur (108) sont fixés de manière à se faire face avec une partie d'ouverture (109) interposée entre eux ; et
un dispositif informatique (111),
dans lequel un deuxième fantôme (303) d'un deuxième matériau de base (203), qui a des parties d'épaisseurs différentes, est installé sur le filtre (105),
le dispositif informatique (111) est configuré pour déplacer le filtre (105) en utilisant le mécanisme d'entraînement de filtre (304) de sorte que des rayons X (104) provenant du tube à rayons X (103) soient transmis à travers une partie d'épaisseur souhaitée du deuxième fantôme (303), et
le dispositif informatique (111) est configuré pour irradier des rayons X (104) provenant du tube à rayons X (103) dans un état dans lequel un premier fantôme (301, 401) d'un premier matériau de base (202), qui a un coefficient d'atténuation linéaire plus petit que le deuxième matériau de base (203), est inséré dans la partie d'ouverture (109) de sorte que des rayons X (104) provenant du tube à rayons X (103) soient transmis à travers le premier fantôme (301, 401) du premier matériau de base (202), et est configuré pour acquérir des données de projection sur la base d'une valeur de détection émise par le détecteur (108).

2. Appareil de tomodensitométrie à rayons X à comptage de photons (101) selon la revendication 1,
dans lequel le dispositif informatique (111) est configuré pour acquérir les données de projection pour chaque combinaison d'une pluralité d'épaisseurs du premier matériau de base (202) et d'une pluralité d'épaisseurs du deuxième matériau de base (203), et est configuré pour obtenir des données d'étalonnage (204) du détecteur (108).

3. Appareil de tomodensitométrie à rayons X à comptage de photons (101) selon la revendication 2,
dans lequel le deuxième fantôme (303) est un empilement du deuxième matériau de base (203) ayant la même épaisseur, et
une différence d'épaisseur du deuxième fantôme (303) est due au nombre de couches empilées du deuxième matériau de base (203).

4. Appareil de tomodensitométrie à rayons X à comptage de photons (101) selon la revendication 2,
dans lequel le premier fantôme (301, 401) est un empilement du premier matériau de base (202) ayant la même épaisseur, et
une différence d'épaisseur du premier fantôme (301, 401) est due au nombre de couches empilées du premier matériau de base (202).

5. Appareil de tomodensitométrie à rayons X à comptage de photons (101) selon la revendication 2,
dans lequel le premier fantôme (301, 401) est un premier matériau de base (202) de forme de colonne et est inséré dans la partie d'ouverture (109) de manière à ne pas chevaucher un centre de rotation du portique (102), et
le dispositif informatique (111) est configuré pour acquérir les données de projection pendant que le portique (102) est entraîné en rotation.

6. Appareil de tomodensitométrie à rayons X à comptage de photons (101) selon la revendication 5,
dans lequel une section transversale du premier fantôme (301, 401) est circulaire ou polygonale.

7. Appareil de tomodensitométrie à rayons X à comptage de photons (101) selon la revendication 1,
dans lequel le filtre (105) est un filtre en nœud papillon.

8. Procédé d'acquisition de données d'étalonnage d'un appareil de tomodensitométrie à rayons X à comptage de photons (101) incluant un tube à rayons X (103), un filtre (105), un mécanisme d'entraînement de filtre (304) qui entraîne le filtre (105), un détecteur (108) qui inclut un élément de détection de type comptage de photons, un portique (102) auquel le tube à rayons X (103) et le mécanisme d'entraînement de filtre (304), et le détecteur (108) sont fixés de manière à se faire face avec une partie d'ouverture (109) interposée entre eux, et un dispositif informatique (111), le procédé d'acquisition de données d'étalonnage comprenant :
installer un deuxième fantôme (303) d'un deuxième matériau de base (203), qui a des parties d'épaisseurs différentes, sur le filtre (105) ;
amener le dispositif informatique (111) à déplacer le filtre (105) en utilisant le mécanisme d'entraînement de filtre (304) de sorte que des rayons X (104) provenant du tube à rayons X (103) soient transmis à travers une partie d'épaisseur souhaitée du deuxième fantôme (303) ; et
amener le dispositif informatique (111) à irradier des rayons X (104) à partir du tube à rayons X (103) dans un état dans lequel un premier fantôme (301, 401) d'un premier matériau de base (202), qui a un coefficient d'atténuation linéaire plus petit que le deuxième matériau de base (203), est inséré dans la partie d'ouverture (109) de sorte que des rayons X (104) provenant du tube à rayons X (103) soient transmis à travers le premier fantôme (301, 401) du premier matériau de base (202), et à acquérir des données de projection sur la base d'une valeur de détection émise par le détecteur (108).

9. Procédé d'acquisition de données d'étalonnage selon la revendication 8,
dans lequel le premier fantôme (301, 401) est un premier matériau de base (202) de forme de colonne et est inséré dans la partie d'ouverture (109) de manière à ne pas chevaucher un centre de rotation du portique (102), et
le dispositif informatique (111) acquiert les données de projection pendant que le portique (102) est entraîné en rotation.

10. Procédé d'acquisition de données d'étalonnage selon la revendication 9,
dans lequel une section transversale du premier fantôme (301, 401) est circulaire ou polygonale.
